(19) Europäisches Patentamt
European Patent Office
Office européen des brevets

(11) EP 1 466 981 A1

(12) **DEMANDE DE BREVET EUROPEEN**

(43) Date de publication:
**13.10.2004 Bulletin 2004/42**

(51) Int Cl.[7]: **C12N 15/56**, C12N 9/24, C12N 9/38

(21) Numéro de dépôt: **04291327.7**

(22) Date de dépôt: **06.10.1997**

(84) Etats contractants désignés:
**AT BE CH DE DK ES FI FR GB GR IE IT LI LU MC NL PT SE**

(30) Priorité: **07.10.1996 FR 9612204**

(62) Numéro(s) de document de la (des) demande(s) initiale(s) en application de l'article 76 CBE:
**97943947.8 / 0 954 567**

(71) Demandeur: **Laboratoires Goemar**
**35400 Saint-Malo (FR)**

(72) Inventeurs:
- **Barbeyron, Tristan**
**29233 Cledder (FR)**
- **Potin, Philippe**
**29680 Roscoff (FR)**
- **Richard, Christophe**
**29400 Plougourvest (FR)**
- **Henrissat, Bernard**
**38400 Uriage (FR)**
- **Yvin, Jean-Claude**
**35400 Saint Malo (FR)**
- **Kloareg, Bernard**
**29250 Saint Pol de Leon (FR)**

(74) Mandataire: **Gillard, Marie-Louise et al**
**Cabinet Beau de Loménie**
**158, rue de l'Université**
**75340 Paris Cédex 07 (FR)**

Remarques:
Cette demande a été déposée le 26.05.2004 comme demande divisionnaire de la demande mentionnée sous le code INID 62.

(54) **Gènes de glycosyle hydrolases et leur utilisation pour la production d'enzymes de biodégradation des carraghénanes**

(57) La présente invnetion a pour objet des gènes qui codent pour des glycosyle hydrolases ayant un score HCA avec la kappa-carraghénase d'*Alteromonas carrageenovora* qui est supérieur ou égal à 75 % sur le domaine s'étendant entre les acides aminés 117 et 262 de la séquence protéique SEQ ID N° 2 de ladite kappa-carraghénase.

EP 1 466 981 A1

LISTE DE SEQUENCES

(1) INFORMATIONS GENERALES:

(i) DEPOSANT:
(A) NOM: LABORATOIRES GOEMAR S.A.
(B) RUE: La Madeleine B.P. 55
(C) VILLE: Saint-Malo
(E) PAYS: France
(F) CODE POSTAL: 35413 Cedex
(G) TELEPHONE: 99 21 53 70
(H) TELECOPIE: 99 82 56 17

(ii) TITRE DE L' INVENTION: Gènes de glycolyse hydrolases et leur utilisation pour la production d'enzymes de biodégradation des carraghénanes

(iii) NOMBRE DE SEQUENCES: 4

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:
(A) LONGUEUR: 2180 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(ix) CARACTERISTIQUE:
(A) NOM/CLE: CDS
(B) EMPLACEMENT:join(1..498, 741..1940, 2009..2179)

(xi) DESCRIPTION DE LA SEQUENCE: SEQ ID NO: 1:

```
GAT CAT ATC ATT CCT TTG CAA ATT AAA AAT TCT CAA GAT AGT CAA ATA        48
Asp His Ile Ile Pro Leu Gln Ile Lys Asn Ser Gln Asp Ser Gln Ile
  1               5                  10                  15
```

```
ATT AGT TTT TTT AAA GCT GAC AAA GGG AGT GTG AGC AGG CAA GTA CAC       96
Ile Ser Phe Phe Lys Ala Asp Lys Gly Ser Val Ser Arg Gln Val His
                20                  25                  30

CCA CCT TGG CCT GTG CCT TGT AAA AGT AAA CTG CAA GAG CAA GAT AGT      144
Pro Pro Trp Pro Val Pro Cys Lys Ser Lys Leu Gln Glu Gln Asp Ser
            35                  40                  45

AGT GAG TCT AAA GAG AGT AAG GCA GAG CAA GTT AAA ATT AAC AAC TGC      192
Ser Glu Ser Lys Glu Ser Lys Ala Glu Gln Val Lys Ile Asn Asn Cys
        50                  55                  60

GTT GTA CAG AAC GCA ATG CTG TAC ATA GAA AAC AAT TAT TTC AAC GAT      240
Val Val Gln Asn Ala Met Leu Tyr Ile Glu Asn Asn Tyr Phe Asn Asp
 65                  70                  75                  80

ATA AAT ATA GAC ACG GTT GCT TTT TCT GTT GGC GTA AGT CGC TCT TAT      288
Ile Asn Ile Asp Thr Val Ala Phe Ser Val Gly Val Ser Arg Ser Tyr
                 85                  90                  95

CTC GTT AAA CAA TTT AAG TTA GCA ACG AAT AAA ACG ATT AAT AAT AGA      336
Leu Val Lys Gln Phe Lys Leu Ala Thr Asn Lys Thr Ile Asn Asn Arg
            100                 105                 110

ATC ATA GAA GTA AGA ATA GAG CAG GCT AAA AAA GTA TTA CTA AAA AAA      384
Ile Ile Glu Val Arg Ile Glu Gln Ala Lys Lys Val Leu Leu Lys Lys
        115                 120                 125

TCT GTT ACA GAA ACA GCT TAT GAA GTT GGT TTT AAT AAC TCA AAC TAC      432
Ser Val Thr Glu Thr Ala Tyr Glu Val Gly Phe Asn Asn Ser Asn Tyr
    130                 135                 140

TTC GCG ACA GTT TTT AAA AAA AGA ACA AAC TAC ACG CCC AAG CAA TTT      480
Phe Ala Thr Val Phe Lys Lys Arg Thr Asn Tyr Thr Pro Lys Gln Phe
145                 150                 155                 160

AAA CGT ACT TTT TCC AGC TAAAACTACA ACTAAATAAC GATTAAAAGC             528
Lys Arg Thr Phe Ser Ser
                165

CATTTTTAGA GAACAGTAAA ACCATTTTTT GAGGTTTGGT GTTGTATATA AATATTAAAT    588
```

```
ATCCCCACTC GCTCAGCTTT TTTTGTGCGA GTTGTGAGAA TTAGCTTAAC AGGTAAGGTT    648
TACGTATCTG TATATCTAAA CTCTTCGAAT ATAACACTGT ATCTGTTGCT GAGCTGTGGC    708
TCAGTTCACA CTAACAAAGG ATGGATAAAT AA ATG AAA CCT ATA AGT ATT GTG      761
                                    Met Lys Pro Ile Ser Ile Val
                                                170

GCA TTC CCT ATA CCA GCT ATA AGT ATG CTT CTT TTA AGT GCA GTA TCA      809
Ala Phe Pro Ile Pro Ala Ile Ser Met Leu Leu Leu Ser Ala Val Ser
    175                 180                 185

CAA GCA GCA TCT ATG CAA CCT CCC ATC GCA AAA CCT GGT GAA ACA TGG      857
Gln Ala Ala Ser Met Gln Pro Pro Ile Ala Lys Pro Gly Glu Thr Trp
190                 195                 200                 205

ATT TTA CAA GCC AAA CGC TCT GAC GAA TTT AAC GTA AAA GAT GCG ACA      905
Ile Leu Gln Ala Lys Arg Ser Asp Glu Phe Asn Val Lys Asp Ala Thr
                210                 215                 220

AAG TGG AAC TTT CAA ACA GAA AAC TAT GGG GTA TGG TCT TGG AAA AAT      953
Lys Trp Asn Phe Gln Thr Glu Asn Tyr Gly Val Trp Ser Trp Lys Asn
            225                 230                 235

GAA AAT GCG ACA GTA TCT AAT GGC AAA CTA AAA TTA ACC ACT AAG CGA     1001
Glu Asn Ala Thr Val Ser Asn Gly Lys Leu Lys Leu Thr Thr Lys Arg
        240                 245                 250

GAA TCT CAT CAA CGT ACA TTC TGG GAT GGC TGT AAT CAG CAG CAA GTT     1049
Glu Ser His Gln Arg Thr Phe Trp Asp Gly Cys Asn Gln Gln Gln Val
    255                 260                 265

GCA AAT TAC CCA CTT TAT TAT ACA TCG GGT GTC GCT AAA TCC AGA GCT     1097
Ala Asn Tyr Pro Leu Tyr Tyr Thr Ser Gly Val Ala Lys Ser Arg Ala
270                 275                 280                 285

ACA GGT AAT TAT GGC TAT TAC GAA GCT CGA ATC AAA GGA GCG AGT ACA     1145
Thr Gly Asn Tyr Gly Tyr Tyr Glu Ala Arg Ile Lys Gly Ala Ser Thr
                290                 295                 300

TTT CCT GGC GTA TCG CCT GCT TTT TGG ATG TAT AGC ACC ATT GAC CGT     1193
Phe Pro Gly Val Ser Pro Ala Phe Trp Met Tyr Ser Thr Ile Asp Arg
            305                 310                 315

TCA TTA ACG AAA GAA GGG GAT GTC CAA TAT AGC GAA ATA GAC GTA GTG     1241
Ser Leu Thr Lys Glu Gly Asp Val Gln Tyr Ser Glu Ile Asp Val Val
        320                 325                 330
```

```
GAA CTT ACT CAA AAA AGT GCA GTG AGA GAG TCT GAT CAT GAC TTA CAC        1289
Glu Leu Thr Gln Lys Ser Ala Val Arg Glu Ser Asp His Asp Leu His
    335                 340                 345

AAT ATT GTA GTA AAA AAT GGA AAA CCA ACA TGG ATG CGT CCA GGG TCT        1337
Asn Ile Val Val Lys Asn Gly Lys Pro Thr Trp Met Arg Pro Gly Ser
350                 355                 360                 365

TTT CCG CAG ACA AAT CAT AAC GGA TAC CAT CTA CCT TTC GAT CCT CGA        1385
Phe Pro Gln Thr Asn His Asn Gly Tyr His Leu Pro Phe Asp Pro Arg
                370                 375                 380

AAT GAC TTT CAC ACC TAT GGT GTC AAT GTA ACT AAA GAC AAG ATC ACT        1433
Asn Asp Phe His Thr Tyr Gly Val Asn Val Thr Lys Asp Lys Ile Thr
            385                 390                 395

TGG TAC GTA GAT GGT GAA ATT GTG GGC GAA AAG GAT AAC TTA TAC TGG        1481
Trp Tyr Val Asp Gly Glu Ile Val Gly Glu Lys Asp Asn Leu Tyr Trp
        400                 405                 410

CAT CGT CAA ATG AAT CTC ACA TTA TCA CAA GGC TTA CGC GCG CCG CAT        1529
His Arg Gln Met Asn Leu Thr Leu Ser Gln Gly Leu Arg Ala Pro His
    415                 420                 425

ACA CAA TGG AAA TGT AAT CAA TTT TAC CCA TCA GCG AAT AAA TCA GCA        1577
Thr Gln Trp Lys Cys Asn Gln Phe Tyr Pro Ser Ala Asn Lys Ser Ala
430                 435                 440                 445

GAA GGC TTC CCA ACA TCA ATG GAA GTT GAT TAT GTA AGA ACG TGG GTA        1625
Glu Gly Phe Pro Thr Ser Met Glu Val Asp Tyr Val Arg Thr Trp Val
                450                 455                 460

AAG GTG GGC AAT AAC AAC TCT GCT CCA GGC GAG GGG CAG TCA TGT CCT        1673
Lys Val Gly Asn Asn Asn Ser Ala Pro Gly Glu Gly Gln Ser Cys Pro
            465                 470                 475

AAC ACG TTT GTA GCT GTC AAT AGT GTT CAA CTA AGC GCA GCA AAA CAA        1721
Asn Thr Phe Val Ala Val Asn Ser Val Gln Leu Ser Ala Ala Lys Gln
        480                 485                 490

ACA CTT CGA AAG GGC CAA TCT ACA ACG CTA GAA AGC ACA GTT CTT CCA        1769
Thr Leu Arg Lys Gly Gln Ser Thr Thr Leu Glu Ser Thr Val Leu Pro
    495                 500                 505
```

```
AAC TGT GCA ACC AAC AAG AAA GTC ATT TAT TCA TCA AGC AAT AAA AAT        1817
Asn Cys Ala Thr Asn Lys Lys Val Ile Tyr Ser Ser Ser Asn Lys Asn
510                 515                 520                 525

GTG GCA ACT GTG AAC AGT GCT GGC GTT GTA AAA GCT AAA AAT AAA GGC        1865
Val Ala Thr Val Asn Ser Ala Gly Val Val Lys Ala Lys Asn Lys Gly
                530                 535                 540

ACT GCG ACG ATT ACG GTT AAA ACT AAA AAC AAA GGG AAA ATA GAT AAA        1913
Thr Ala Thr Ile Thr Val Lys Thr Lys Asn Lys Gly Lys Ile Asp Lys
            545                 550                 555

TTA ACC ATT GCG GTG AAT TAAGCTAACT CAAACTAGCC TCGAAGGATT               1961
Leu Thr Ile Ala Val Asn
        560

GAGGCACTTT ATTTATAGGT CTCAGGCTTC GACTTTTTGG AGGGGGT ATG AAA AAG        2017
                                                    Met Lys Lys
                                                        565

GTA AAT TTA TCC AGC AAG TGG ATA ATT AGC ATT AGT TTA CTA ATC ATT        2065
Val Asn Leu Ser Ser Lys Trp Ile Ile Ser Ile Ser Leu Leu Ile Ile
            570                 575                 580

TGT GAT TAT GTT TAT TTA ATA CGA ACA AAC GTT AAC GAG CAA GCT AAC        2113
Cys Asp Tyr Val Tyr Leu Ile Arg Thr Asn Val Asn Glu Gln Ala Asn
        585                 590                 595

GCA GAA GCT ACT GCA CAT ATG CAT TAC AAA ATA AAT AAT ACG AAA CAC        2161
Ala Glu Ala Thr Ala His Met His Tyr Lys Ile Asn Asn Thr Lys His
    600                 605                 610

TCA AAA GGA AAG CTT GAT C                                               2180
Ser Lys Gly Lys Leu Asp
615                 620
```

(2) INFORMATIONS POUR LA SEQ ID NO: 2:

(i) CARACTERISTIQUES DE LA SEQUENCE:
(A) LONGUEUR: 620 acides aminés
(B) TYPE: acide aminé
(D) CONFIGURATION: linéaire

(ii) TYPE DE MOLECULE: protéine

(xi) DESCRIPTION DE LA SEQUENCE: SEQ ID NO: 2:

```
Asp His Ile Ile Pro Leu Gln Ile Lys Asn Ser Gln Asp Ser Gln Ile
  1               5                  10                  15
Ile Ser Phe Phe Lys Ala Asp Lys Gly Ser Val Ser Arg Gln Val His
             20                  25                  30
Pro Pro Trp Pro Val Pro Cys Lys Ser Lys Leu Gln Glu Gln Asp Ser
         35                  40                  45
Ser Glu Ser Lys Glu Ser Lys Ala Glu Gln Val Lys Ile Asn Asn Cys
     50                  55                  60
Val Val Gln Asn Ala Met Leu Tyr Ile Glu Asn Asn Tyr Phe Asn Asp
 65                  70                  75                  80
Ile Asn Ile Asp Thr Val Ala Phe Ser Val Gly Val Ser Arg Ser Tyr
                 85                  90                  95
Leu Val Lys Gln Phe Lys Leu Ala Thr Asn Lys Thr Ile Asn Asn Arg
            100                 105                 110
Ile Ile Glu Val Arg Ile Glu Gln Ala Lys Lys Val Leu Leu Lys Lys
        115                 120                 125
Ser Val Thr Glu Thr Ala Tyr Glu Val Gly Phe Asn Asn Ser Asn Tyr
    130                 135                 140
Phe Ala Thr Val Phe Lys Lys Arg Thr Asn Tyr Thr Pro Lys Gln Phe
145                 150                 155                 160
Lys Arg Thr Phe Ser Ser Met Lys Pro Ile Ser Ile Val Ala Phe Pro
                165                 170                 175
Ile Pro Ala Ile Ser Met Leu Leu Leu Ser Ala Val Ser Gln Ala Ala
            180                 185                 190
Ser Met Gln Pro Pro Ile Ala Lys Pro Gly Glu Thr Trp Ile Leu Gln
        195                 200                 205
Ala Lys Arg Ser Asp Glu Phe Asn Val Lys Asp Ala Thr Lys Trp Asn
    210                 215                 220
Phe Gln Thr Glu Asn Tyr Gly Val Trp Ser Trp Lys Asn Glu Asn Ala
225                 230                 235                 240
Thr Val Ser Asn Gly Lys Leu Lys Leu Thr Thr Lys Arg Glu Ser His
                245                 250                 255
Gln Arg Thr Phe Trp Asp Gly Cys Asn Gln Gln Gln Val Ala Asn Tyr
            260                 265                 270
Pro Leu Tyr Tyr Thr Ser Gly Val Ala Lys Ser Arg Ala Thr Gly Asn
        275                 280                 285
Tyr Gly Tyr Tyr Glu Ala Arg Ile Lys Gly Ala Ser Thr Phe Pro Gly
    290                 295                 300
Val Ser Pro Ala Phe Trp Met Tyr Ser Thr Ile Asp Arg Ser Leu Thr
305                 310                 315                 320
Lys Glu Gly Asp Val Gln Tyr Ser Glu Ile Asp Val Val Glu Leu Thr
                325                 330                 335
Gln Lys Ser Ala Val Arg Glu Ser Asp His Asp Leu His Asn Ile Val
            340                 345                 350
```

```
Val Lys Asn Gly Lys Pro Thr Trp Met Arg Pro Gly Ser Phe Pro Gln
        355                 360                 365
Thr Asn His Asn Gly Tyr His Leu Pro Phe Asp Pro Arg Asn Asp Phe
    370                 375                 380
His Thr Tyr Gly Val Asn Val Thr Lys Asp Lys Ile Thr Trp Tyr Val
385                 390                 395                 400
Asp Gly Glu Ile Val Gly Glu Lys Asp Asn Leu Tyr Trp His Arg Gln
                405                 410                 415
Met Asn Leu Thr Leu Ser Gln Gly Leu Arg Ala Pro His Thr Gln Trp
            420                 425                 430
Lys Cys Asn Gln Phe Tyr Pro Ser Ala Asn Lys Ser Ala Glu Gly Phe
        435                 440                 445
Pro Thr Ser Met Glu Val Asp Tyr Val Arg Thr Trp Val Lys Val Gly
    450                 455                 460
Asn Asn Asn Ser Ala Pro Gly Glu Gly Gln Ser Cys Pro Asn Thr Phe
465                 470                 475                 480
Val Ala Val Asn Ser Val Gln Leu Ser Ala Ala Lys Gln Thr Leu Arg
                485                 490                 495
Lys Gly Gln Ser Thr Thr Leu Glu Ser Thr Val Leu Pro Asn Cys Ala
            500                 505                 510
Thr Asn Lys Lys Val Ile Tyr Ser Ser Ser Asn Lys Asn Val Ala Thr
        515                 520                 525
Val Asn Ser Ala Gly Val Val Lys Ala Lys Asn Lys Gly Thr Ala Thr
    530                 535                 540
Ile Thr Val Lys Thr Lys Asn Lys Gly Lys Ile Asp Lys Leu Thr Ile
545                 550                 555                 560
Ala Val Asn Met Lys Lys Val Asn Leu Ser Ser Lys Trp Ile Ile Ser
                565                 570                 575
Ile Ser Leu Leu Ile Ile Cys Asp Tyr Val Tyr Leu Ile Arg Thr Asn
            580                 585                 590
Val Asn Glu Gln Ala Asn Ala Glu Ala Thr Ala His Met His Tyr Lys
        595                 600                 605
Ile Asn Asn Thr Lys His Ser Lys Gly Lys Leu Asp
    610                 615                 620
```

(2) INFORMATIONS POUR LA SEQ ID NO: 3:

(i) CARACTERISTIQUES DE LA SEQUENCE:

(A) LONGUEUR: 2600 paires de bases

(B) TYPE: nucléotide

(C) NOMBRE DE BRINS: simple

(D) CONFIGURATION: linéaire

(ii) TYPE DE MOLECULE: ADN (génomique)

(iii) HYPOTHETIQUE: NON

(ix) CARACTERISTIQUE:
(A) NOM/CLE: CDS
(B) EMPLACEMENT:875..2509

(xi) DESCRIPTION DE LA SEQUENCE: SEQ ID NO: 3:

```
GCCTCCGTAT TCGACAATGT TGTACGATGC TTGGCGATTC GGACTCTGTT TAAGCACTCG     60
ATTTCGTAAA GGCACTATCC ACTCATTCAT TCCGACTCAA TATTCTTTTC GACAAATGCA    120
ACCGGTTCCA TTGAAAAGGC CCTAAAAATA CAGCTTTCCC GCCCCCCATC GTAGAAGGTT    180
CCAATATGCT TCAACCCCTT TTTCAGCCTT ACTTCAGGGG TATTACTTTC ATGCCTAGGG    240
CCGCAAATAC ATTCGCTTGG ACCCAGTCAC CTATATAATT GAATACGGAA CTACCCATGG    300
CTTCCTTCCC TTTGGGAACC TATGGTACAG ACTTGCCTTT TTTAAACCGG TTACTTCAGC    360
TAATTCGCCA AGCTGGTTCC TTCATAACCT TTGGCCCGAA ACACCTTGCA AGCACATAAA    420
TCTTATCCAA TATTTTGCGG TCTCATGGGA CAAATCTATA ACAAACATTC AATTTTACCA    480
AACGTTCGGT AATAAATCTA GTCAAAAACG GGGTCCGATT CATTTTAGAA GAAAGGTAAA    540
GCCCCCAAAA GAGCGGTTTA CTTGAAGATA TGATTTATAA AACACAATAA GTGACAAAGG    600
AAGATCATGG CTATAATTAG TTGAAAAAAC AGGGCTTACC ATGACATGGA GCTTTATTGA    660
AAACAGATGT CCAACAAGAA TAAAGGAGGG CCGTTCGACC GCGACGTTTA AATAAAAACA    720
TATTCCATAT CAAAATTTAA TTAAGGTTCT TTCCTACAGT ATTTATAAGA AATTACTAAA    780
ATTAGTTAGG ATAATACTAC AAAATGGTAA AATTGGATTA CTCAGATTGA ACCATAGCCT    840
CTACTTTAGT CGGCTAACAA AAACAATTAT AGTA ATG AAA AAA CCA AAT TTT         892
                                      Met Lys Lys Pro Asn Phe
                                        1               5

TAT GGC AAG ATG GGT AGA ACT GCA CTT TCA AGT CTT TTC TAC CTC TTT       940
Tyr Gly Lys Met Gly Arg Thr Ala Leu Ser Ser Leu Phe Tyr Leu Phe
            10                  15                  20

TTC CTA GGC CTT GTG TAT GGG CAA CAA CCT ACG AAG ACT TCA AAT CCG       988
Phe Leu Gly Leu Val Tyr Gly Gln Gln Pro Thr Lys Thr Ser Asn Pro
        25                  30                  35

AAC GAT CAG TGG ACC ATC AAA TGG AGT GCT TCG GAC GAA TTC AAC AAA      1036
Asn Asp Gln Trp Thr Ile Lys Trp Ser Ala Ser Asp Glu Phe Asn Lys
    40                  45                  50

AAT GAC CCC GAC TGG GCA AAA TGG ATC AAG ACA GGA AAC CTT CCG AAT      1084
Asn Asp Pro Asp Trp Ala Lys Trp Ile Lys Thr Gly Asn Leu Pro Asn
55                  60                  65                  70

ACA TCG GCA TGG AAA TGG AAC AAT CAA AAA AAC GTA AAG ATT TCC AAC      1132
Thr Ser Ala Trp Lys Trp Asn Asn Gln Lys Asn Val Lys Ile Ser Asn
                75                  80                  85
```

```
GGA ATT GCG GAA CTA ACG ATG AGG CAT AAC GCC AAT AAT ACC CCA CCT      1180
Gly Ile Ala Glu Leu Thr Met Arg His Asn Ala Asn Asn Thr Pro Pro
                90                  95                 100

GAC GGA GGA ACC TAT TTC ACC TCT GGG ATA TTT AAG TCG TAC CAA AAA      1228
Asp Gly Gly Thr Tyr Phe Thr Ser Gly Ile Phe Lys Ser Tyr Gln Lys
        105                 110                 115

TTT ACG TAT GGA TAC TTT GAG GCC AAA ATC CAA GGA GCG GAT ATA GGT      1276
Phe Thr Tyr Gly Tyr Phe Glu Ala Lys Ile Gln Gly Ala Asp Ile Gly
    120                 125                 130

GAA GGC GTA TGC CCA TCG TTT TGG CTT TAT AGT GAT TTC GAC TAT TCC      1324
Glu Gly Val Cys Pro Ser Phe Trp Leu Tyr Ser Asp Phe Asp Tyr Ser
135                 140                 145                 150

GTA GCC AAT GGG GAA ACG GTA TAC AGT GAA ATA GAT GTA GTT GAA CTA      1372
Val Ala Asn Gly Glu Thr Val Tyr Ser Glu Ile Asp Val Val Glu Leu
                155                 160                 165

CAA CAA TTC GAT TGG TAT GAA GGC CAT CAG GAC GAC ATT TAC GAC ATG      1420
Gln Gln Phe Asp Trp Tyr Glu Gly His Gln Asp Asp Ile Tyr Asp Met
            170                 175                 180

GAC TTA AAT CTA CAC GCC GTT GTC AAA GAA AAC GGA CAG GGG GTT TGG      1468
Asp Leu Asn Leu His Ala Val Val Lys Glu Asn Gly Gln Gly Val Trp
        185                 190                 195

AAA AGG CCA AAA ATG TAC CCT CAA GAA CAG TTG AAC AAA TGG AGA GCC      1516
Lys Arg Pro Lys Met Tyr Pro Gln Glu Gln Leu Asn Lys Trp Arg Ala
    200                 205                 210

ATG GAC CCG AGT AAA GAC TTT CAT ATC TAT GGT TGT GAA GTG AAC CAG      1564
Met Asp Pro Ser Lys Asp Phe His Ile Tyr Gly Cys Glu Val Asn Gln
215                 220                 225                 230

AAC GAA ATC ATA TGG TAT GTT GAC GGT GTC GAG GTT GCC CGA AAA CCA      1612
Asn Glu Ile Ile Trp Tyr Val Asp Gly Val Glu Val Ala Arg Lys Pro
                235                 240                 245

AAT AAA TAT TGG CAT CGC CCC ATG AAC GTT ACC CTT TCA TTG GGA CTC      1660
Asn Lys Tyr Trp His Arg Pro Met Asn Val Thr Leu Ser Leu Gly Leu
            250                 255                 260
```

```
AGA AAA CCA TTT GTG AAA TTT TTC GAC AAT AAG AAC AAT GCC ATA AAT        1708
Arg Lys Pro Phe Val Lys Phe Phe Asp Asn Lys Asn Asn Ala Ile Asn
        265                 270                 275

CCA GAA ACC GAT GCC AAG GCA AGG GAA AAA TTA TCG GAT ATA CCT ACA        1756
Pro Glu Thr Asp Ala Lys Ala Arg Glu Lys Leu Ser Asp Ile Pro Thr
    280                 285                 290

TCG ATG TAT GTG GAT TAC GTT CGG GTC TGG GAA AAA TCA GCA GGT AAC        1804
Ser Met Tyr Val Asp Tyr Val Arg Val Trp Glu Lys Ser Ala Gly Asn
295                 300                 305                 310

ACT ACC AAT CCC CCA ACC AGC GAG GTC GGC ACA CTA AAA ACA AAG GGT        1852
Thr Thr Asn Pro Pro Thr Ser Glu Val Gly Thr Leu Lys Thr Lys Gly
                315                 320                 325

TCG AAA CTG GTG ATT GAC CAT TGG GAT GCA AGT ACA GGG ACT ATT TCG        1900
Ser Lys Leu Val Ile Asp His Trp Asp Ala Ser Thr Gly Thr Ile Ser
            330                 335                 340

GCT GTC AGT AAC AAT ACA AAG ACA GGT CAA TAT GCC GGT TCA GTG AAC        1948
Ala Val Ser Asn Asn Thr Lys Thr Gly Gln Tyr Ala Gly Ser Val Asn
        345                 350                 355

AAC GCG AGC ATC GCC CAG ATA GTA ACA TTA AAA GCG AAT ACT TCA TAT        1996
Asn Ala Ser Ile Ala Gln Ile Val Thr Leu Lys Ala Asn Thr Ser Tyr
    360                 365                 370

AAG GTA TCG GCT TTC GGA AAG GCC AGC TCA CCC GGA ACA TCG GCT TAT        2044
Lys Val Ser Ala Phe Gly Lys Ala Ser Ser Pro Gly Thr Ser Ala Tyr
375                 380                 385                 390

CTA GGC ATT AGT AAA GCA TCC AAC AAC GAA CTC ATA AGC AAT TTT GAA        2092
Leu Gly Ile Ser Lys Ala Ser Asn Asn Glu Leu Ile Ser Asn Phe Glu
                395                 400                 405

TTC AAA ACA ACC TCA TAC TCC AAA GGC GAG ATT GAG ATA AGA ACT GGA        2140
Phe Lys Thr Thr Ser Tyr Ser Lys Gly Glu Ile Glu Ile Arg Thr Gly
            410                 415                 420

AAT GTT CAG GAA TCA TAT CGC ATA TGG TAT TGG TCT TCC GGG CAA GCC        2188
Asn Val Gln Glu Ser Tyr Arg Ile Trp Tyr Trp Ser Ser Gly Gln Ala
        425                 430                 435
```

```
TAT TGC GAT GAT TTT AAC CTT GTT GAA ATA AAC AGC GGG GCT TCA CAA       2236
Tyr Cys Asp Asp Phe Asn Leu Val Glu Ile Asn Ser Gly Ala Ser Gln
    440                 445                 450

CTC AAT GAA AAT GAG ACT GAA ACA GCA CTG GAA AAA GGT ATA CAC ATT       2284
Leu Asn Glu Asn Glu Thr Glu Thr Ala Leu Glu Lys Gly Ile His Ile
455                 460                 465                 470

TAT CCG AAT CCC TAT AAA AAC GGT CCA TTG ACA ATC GAT TTT GGC AAA       2332
Tyr Pro Asn Pro Tyr Lys Asn Gly Pro Leu Thr Ile Asp Phe Gly Lys
                475                 480                 485

CCC TTC AGC GGC GAG GTC CAA ATC ACC GGT TTA AAC GGT AGA ACA TTC       2380
Pro Phe Ser Gly Glu Val Gln Ile Thr Gly Leu Asn Gly Arg Thr Phe
            490                 495                 500

TTA AGA AGA AAT GTT GTC GAT CAA ACT TCG GTT CAG CTC CTA GAA TCC       2428
Leu Arg Arg Asn Val Val Asp Gln Thr Ser Val Gln Leu Leu Glu Ser
        505                 510                 515

AAA TCT AAA TTC AAG AGC GGT CTA TAT ATC GTT AAA ATT AGT GGC CCG       2476
Lys Ser Lys Phe Lys Ser Gly Leu Tyr Ile Val Lys Ile Ser Gly Pro
    520                 525                 530

GAT GGA GAG GTT TCA AAA AAG ATA CTC GTG GAG TAACTAAAAA TCAATTTTTA     2529
Asp Gly Glu Val Ser Lys Lys Ile Leu Val Glu
535                 540                 545

CAGGATTACA GACGGGCAAA GGGATTTTCC TTTGCCCGTT TTTAAAATTA TGGGCGGAAA     2589
CGATTGTTGC G                                                          2600
```

(2) INFORMATIONS POUR LA SEQ ID NO: 4:

(i) CARACTERISTIQUES DE LA SEQUENCE:
(A) LONGUEUR: 545 acides aminés
(B) TYPE: acide aminé
(D) CONFIGURATION: linéaire

(ii) TYPE DE MOLECULE: protéine

(xi) DESCRIPTION DE LA SEQUENCE: SEQ ID NO: 4:

```
Met Lys Lys Pro Asn Phe Tyr Gly Lys Met Gly Arg Thr Ala Leu Ser
  1               5                  10                  15
Ser Leu Phe Tyr Leu Phe Phe Leu Gly Leu Val Tyr Gly Gln Gln Pro
            20                  25                  30
```

Thr Lys Thr Ser Asn Pro Asn Asp Gln Trp Thr Ile Lys Trp Ser Ala
35 40 45
Ser Asp Glu Phe Asn Lys Asn Asp Pro Asp Trp Ala Lys Trp Ile Lys
50 55 60
Thr Gly Asn Leu Pro Asn Thr Ser Ala Trp Lys Trp Asn Asn Gln Lys
65 70 75 80
Asn Val Lys Ile Ser Asn Gly Ile Ala Glu Leu Thr Met Arg His Asn
85 90 95
Ala Asn Asn Thr Pro Pro Asp Gly Gly Thr Tyr Phe Thr Ser Gly Ile
100 105 110
Phe Lys Ser Tyr Gln Lys Phe Thr Tyr Gly Tyr Phe Glu Ala Lys Ile
115 120 125
Gln Gly Ala Asp Ile Gly Glu Gly Val Cys Pro Ser Phe Trp Leu Tyr
130 135 140
Ser Asp Phe Asp Tyr Ser Val Ala Asn Gly Glu Thr Val Tyr Ser Glu
145 150 155 160
Ile Asp Val Val Glu Leu Gln Gln Phe Asp Trp Tyr Glu Gly His Gln
165 170 175
Asp Asp Ile Tyr Asp Met Asp Leu Asn Leu His Ala Val Val Lys Glu
180 185 190
Asn Gly Gln Gly Val Trp Lys Arg Pro Lys Met Tyr Pro Gln Glu Gln
195 200 205
Leu Asn Lys Trp Arg Ala Met Asp Pro Ser Lys Asp Phe His Ile Tyr
210 215 220
Gly Cys Glu Val Asn Gln Asn Glu Ile Ile Trp Tyr Val Asp Gly Val
225 230 235 240
Glu Val Ala Arg Lys Pro Asn Lys Tyr Trp His Arg Pro Met Asn Val
245 250 255
Thr Leu Ser Leu Gly Leu Arg Lys Pro Phe Val Lys Phe Phe Asp Asn
260 265 270
Lys Asn Asn Ala Ile Asn Pro Glu Thr Asp Ala Lys Ala Arg Glu Lys
275 280 285
Leu Ser Asp Ile Pro Thr Ser Met Tyr Val Asp Tyr Val Arg Val Trp
290 295 300
Glu Lys Ser Ala Gly Asn Thr Thr Asn Pro Pro Thr Ser Glu Val Gly
305 310 315 320
Thr Leu Lys Thr Lys Gly Ser Lys Leu Val Ile Asp His Trp Asp Ala
325 330 335
Ser Thr Gly Thr Ile Ser Ala Val Ser Asn Asn Thr Lys Thr Gly Gln
340 345 350
Tyr Ala Gly Ser Val Asn Asn Ala Ser Ile Ala Gln Ile Val Thr Leu
355 360 365
Lys Ala Asn Thr Ser Tyr Lys Val Ser Ala Phe Gly Lys Ala Ser Ser
370 375 380
Pro Gly Thr Ser Ala Tyr Leu Gly Ile Ser Lys Ala Ser Asn Asn Glu
385 390 395 400

```
Leu Ile Ser Asn Phe Glu Phe Lys Thr Thr Ser Tyr Ser Lys Gly Glu
                405                 410                 415
Ile Glu Ile Arg Thr Gly Asn Val Gln Glu Ser Tyr Arg Ile Trp Tyr
            420                 425                 430
Trp Ser Ser Gly Gln Ala Tyr Cys Asp Asp Phe Asn Leu Val Glu Ile
        435                 440                 445
Asn Ser Gly Ala Ser Gln Leu Asn Glu Asn Glu Thr Glu Thr Ala Leu
    450                 455                 460
Glu Lys Gly Ile His Ile Tyr Pro Asn Pro Tyr Lys Asn Gly Pro Leu
465                 470                 475                 480
Thr Ile Asp Phe Gly Lys Pro Phe Ser Gly Glu Val Gln Ile Thr Gly
                485                 490                 495
Leu Asn Gly Arg Thr Phe Leu Arg Arg Asn Val Val Asp Gln Thr Ser
            500                 505                 510
Val Gln Leu Leu Glu Ser Lys Ser Lys Phe Lys Ser Gly Leu Tyr Ile
        515                 520                 525
Val Lys Ile Ser Gly Pro Asp Gly Glu Val Ser Lys Lys Ile Leu Val
    530                 535                 540
Glu
545
```

## Description

La présente invention concerne des gènes de glycosyle hydrolases pour la production biotechnologique d'oligosaccharides, notamment d'oligo-carraghénanes sulfatés, plus particulièrement d'oligo-kappa-carraghénanes par biodégradation des carraghénanes.

Les galactanes sulfatés des Rhodophycées, tels que les agars et les carraghénanes, représentent les polysaccharides majeurs des Rhodophycées et sont très largement utilisés en tant qu'agents gélifiants ou épaississants dans diverses branches d'activité, notamment l'agro-alimentaire. Environ 6 000 tonnes d'agars et 22 000 tonnes de carraghénanes sont extraits annuellement des algues rouges marines à cet effet. Les agars sont commercialement produits par des algues rouges des genres *Gelidium* et *Gracilaria.* Les carraghénanes sont quant à eux largement extraits des genres *Chondrus, Gigartina* et *Eucheuma.*

Les carraghénanes sont constitués par la répétition d'unités de D-galactose alternativement liées par des liaisons $\beta$ 1->4 et $\alpha$ 1->3. Selon le nombre et la position de groupements ester-sulfate sur le disaccharide de répétition de la molécule, on distingue ainsi plusieurs types de carraghénanes à savoir : les kappa-carraghénanes qui possèdent un groupement ester-sulfate, les iota-carraghénanes qui possèdent deux groupements ester-sulfate et les lambda-carraghénanes qui possèdent trois groupements ester-sulfate.

Les propriétés physico-chimiques et les utilisations de ces polysaccharides en tant que gélifiants reposent sur leur capacité à établir des transitions conformationnelles pelote-hélice en fonction de l'environnement thermique et ionique [Kloareg et al. Oceanography and Marine Biology - An annual review 26 : 259-315 (1988)].

Par ailleurs, les carraghénanes sont des analogues structuraux des polysaccharides sulfatés de la matrice extracellulaire animale (héparine, chondroïtine, kératane, dermatane) et ils présentent des activités biologiques qui s'apparentent à certaines fonctions de ces glycosaminoglycanes.

En particulier, les carraghénanes sont connus :

(i) - pour leur action sur le système immunitaire en provoquant la sécrétion d'interleukine ou de prostaglandines,
(ii) - pour leur action antivirale sur les virus du sida HIV1, de l'herpès HSV1 et de l'hépatite A,
(iii) - en tant qu'antagonistes de la fixation des facteurs de croissance des cellules humaines et aussi,
(iv) - pour leur action sur la prolifération des kératinocytes et leur action sur le pouvoir contractile des fibroblastes.

Par ailleurs, les oligocarraghénanes agissent sur l'adhérence, la division et la synthèse protéique de cultures de cellules humaines, sans doute en tant qu'analogues structuraux de la partie glycosylée des protéines de la matrice extracellulaire. Dans les plantes, les oligocarraghénanes élicitent très significativement des activités enzymatiques marqueurs de la croissance (amylase) ou du métabolisme phénolique de défense (laminarinase, phénylalanine-ammonium lyase).

Les carraghénanes sont extraits des algues rouges marines par des procédés classiques, tels que l'extraction aqueuse à chaud et les oligo-carraghénanes sont obtenus à partir de carraghénanes par hydrolyse chimique ou de préférence par hydrolyse enzymatique.

La production d'oligo-carraghénanes par hydrolyse enzymatique comprend généralement les étapes de :

1) production d'une glycosyle hydrolase par culture d'une bactérie marine ;
2) hydrolyse enzymatique du carraghénane par la glycosyle hydrolase ainsi obtenue ;
3) fractionnement et purification des oligo-carraghénanes obtenus.

Des microorganismes produisant des enzymes capables d'hydrolyser les iota- et kappa-carraghénanes ont été isolées par Bellion et al. en 1982 [Can. J. Microbiol. **28 :** 874-80, (1982)]. Certaines sont spécifiques pour le $\kappa$- ou le $\iota$ -carraghénane et d'autres sont capables d'hydrolyser les deux substrats. Un autre groupe de bactéries capables de dégrader les carraghénanes a été caractérisé par Sarwar et al. en 1983 [J. Gen. Appl. Microbiol. **29 :** 145-55, (1983)]. Ces bactéries de couleur jaune-orange sont assignées au groupe des bactéries de type *Cytophaga* et certaines de ces bactéries ont la propriété d'hydrolyser à la fois l'agar et les carraghénanes.

La purification et la caractérisation de plusieurs $\iota$ -carraghénases et $\kappa$-carraghénases, telles que les $\iota$ -carraghénase et $\kappa$-carraghénase de *Cytophaga drobachiensis,* la $\iota$ -carraghénase de *Alteromonas fortis* et la $\kappa$-carraghénase de *Alteromonas Carrageenovora,* ont été décrites dans la thèse de P. Potin ["Recherche, production, purification et caractérisation de galactane-hydrolases pour la préparation des parois d'algues rouges", (février 1992)]. Une étude approfondie de la $\kappa$-carraghénase de *Alteromonas Carrageenovora* a été décrite par Potin et al. [Eur. J. Biochem 228, 971-975 (1995)]."

La production d'oligo-carraghénanes pourrait être nettement améliorée si l'on disposait d'enzymes spécifiques et d'outils pour leur obtention par génie génétique.

La Demanderesse a maintenant trouvé de nouveaux gènes de glycosyle hydrolases, qui permettent d'obtenir de manière spécifique des oligo-kappa-carraghénanes.

Ainsi, la présente invention a pour objet les gènes qui codent pour des glycosyle hydrolases ayant un score HCA avec la kappa-carraghénase *d'Alteromonas carrageenovora* qui est supérieur ou égal à 75 %, de préférence supérieur à 80 %, avantageusement supérieur à 85 %, sur le domaine s'étendant entre les acides aminés 117 et 262 de la séquence [SEQ ID N° 2] de la kappa-carraghénase *d'Alteromonas carrageenovora.*

En particulier, l'invention concerne la séquence nucléique [SEQ ID N°3] qui code pour une kappa-carraghénase ayant un score tel que défini ci-dessus dont la séquence en acides aminés est la séquence [SEQ ID N°4].

Les gènes glycosyle hydrolases de l'invention peuvent aussi être obtenus par le procédé qui consiste à sélectionner des protéines qui ont un score HCA avec la kappa-carraghénase *d'Alteromonas carrageenovora* qui est supérieur ou égal à 75 %, de préférence supérieur à 80 %, avantageusement supérieur à 85 %, sur le domaine s'étendant entre les acides aminés 117 et 262 de la séquence [SEQ ID N°2] de la kappa-carraghénase *d'Alteromonas carrageenovora* et à séquencer les gènes ainsi obtenus selon les techniques classiques bien connues de l'homme du métier.

Enfin, la présente invention concerne l'utilisation des gènes de glycosyle hydrolases ci-dessus pour l'obtention de glycosyle hydrolases par génie génétique, lesquelles sont utiles pour la production biotechnologique d'oligo-carraghénanes.

Les glysosyle hydrolases selon l'invention sont donc caractérisées par le score HCA qu'elles possèdent avec un domaine particulier de la séquence en acides aminés de la kappa-carraghénase *d'Alteromonas carrageenovora.*

La méthode HCA de l'anglais "Hydrophobic Cluster Analysis" est une méthode d'analyse des séquences de protéines représentées en structure bidimensionnelle, qui a été décrite par Gaboriaud et al. [FEBS Letters 224 149-155 (1987)].

On sait que la structure tridimensionnelle d'une protéine conditionne ses propriétés biologiques, la production d'une protéine active exigeant un repliement correct.

On sait aussi que la structure primaire de protéines varie de façon beaucoup plus importante que les structures d'ordre supérieur et que les protéines peuvent être regroupées en familles montrant des structures secondaires et tertiaires similaires ayant parfois des séquences primaires si divergentes que la parenté de telles protéines entre elles n'est pas évidente. Le code reliant structure primaire et structure secondaire apparaît donc très dégénéré puisque des structures primaires très différentes peuvent aboutir à des structures secondaires et tertiaires similaires [Structure 3, 853-859 (1995) et Proc. Natl. Acad. Sci. USA 92 (1995)].

L'utilisation de la méthode HCA a montré que la répartition, la taille et la forme de ces amas hydrophobes le long des séquences d'acides aminés sont représentatifs du repliement 3D des protéines étudiées.

De plus, Woodcock et al. [Protein. Eng. 5, 629-635, (1992)] ont démontré que les amas hydrophobes définis par le diagramme 2D α-hélicoïdal sont statistiquement centrés sur les structures secondaires régulières (hélices α, brins β), que le diagramme 2D basé sur l'hélice α porte la plus grande quantité d'information structurale et que la correspondance entre amas hydrophobes et éléments de structure secondaire est de même qualité pour tout type de repliement (tout α, tout β, α/β et α + β), démontrant ainsi que la méthode HCA peut être utilisée quel que soit le type de protéine.

L. Lemesle-Varloot et al. [Biochimie 72, 555-574, (1990)] ont montré que lorsque deux protéines présentent une distribution similaire des amas hydrophobes sur un domaine d'au moins 50 résidus, leurs structures tridimensionnelles dans ce domaine sont considérées comme superposables et leurs fonctions analogues.

Ainsi, par exemple, Barbeyron et al. [Gene 139, 105-109 (1994)] ont utilisé cette méthode HCA pour comparer les similarités de forme, de distribution et de taille de plusieurs amas hydrophobes de la κ-carraghénase *d'Alteromonas Carrageenovora* par rapport aux enzymes de la famille 16 des glycosyle hydrolases.

La représentation bidimensionnelle utilisée dans la méthode HCA est une hélice α où, par traitement informatique, les acides aminés sont disposés à raison de 3,6 résidus par révolution. Afin d'obtenir une image plane facile à lire, l'hélice est coupée dans le sens longitudinal. Enfin, pour obtenir dans leur intégralité les amas hydrophobes qui sont situés sur les bords de l'image, le diagramme est dupliqué. La méthode utilise un code qui ne reconnaît que deux états : l'état hydrophobe et l'état hydrophile.

Les acides aminés reconnus comme étant hydrophobes sont identifiés et regroupés en figures géométriques caractéristiques. L'utilisation de ces deux états permet de s'affranchir de la tolérance que montrent les structures bi- et tridimensionnelles vis-à-vis de la variabilité des séquences primaires. De plus, cette représentation permet d'observer rapidement des interactions sur une courte ou une moyenne distance puisque le premier acide aminé et le second acide aminé voisin d'un résidu donné sont localisés sur un segment de 17 acides aminés. Enfin, aucune "fenêtre" de longueur prédéfinie n'est utilisée, contrairement aux méthodes d'analyses basées sur les structures primaires ou secondaires des protéines.

La caractéristique fondamentale de la représentation en hélice α est que, pour une protéine globulaire donnée ou seulement un domaine de cette protéine, la distribution des résidus hydrophobes sur le diagramme n'est pas aléatoire. Les résidus hydrophobes (VILFWMY) forment des amas de géométrie et de taille variées. Sur le diagramme, les faces hydrophiles et hydrophobes des hélices amphiphiles sont très reconnaissables. Ainsi, un amas en diamant horizontal correspond à la face hydrophobe d'une hélice α, les hélices internes apparaissent comme de grands amas hydrophobes horizontaux et les brins β comme des amas hydrophobes assez courts et verticaux. La méthode permet d'identifier les résidus hydrophobes formant le coeur des protéines globulaires et de localiser les éléments de structure secondaire que sont les hélices α et les brins β, indépendamment de toute connaissance de la structure secondaire de la protéine étudiée.

Le score HCA entre deux protéines se calcule de la façon suivante :

pour chaque amas :

$$\text{score HCA} = 2CR/(RC_1 + RC_2) \times 100~\%$$

où

- $RC_1$ et $RC_2$ représentent respectivement le nombre de résidus hydrophobes dans l'amas de la protéine 1 (amas 1) et l'amas de la protéine 2 (amas 2).
- CR représente le nombre de résidus hydrophobes dans l'amas 1 qui sont en correspondance avec les résidus hydrophobes dans l'amas 2.

La valeur moyenne obtenue pour l'ensemble des amas le long des séquences protéiques comparées donne le score final HCA.

Sur les profils HCA, les acides aminés sont représentés par leur code standard à une seule lettre à l'exception de la proline (P), la glycine (G), la sérine (S), la thréonine (T).

En effet, du fait de leurs propriétés particulières, ces résidus sont représentés par les symboles spéciaux indiqués ci-après afin de faciliter leur identification visuelle sur les diagrammes HCA (voir liste des abréviations).

La proline introduit de fortes contraintes dans la chaîne polypeptidique et est considérée de manière systématique comme une interruption dans les amas. En effet, les résidus proline stoppent ou déforment les hélices et les feuillets. La glycine possède une flexibilité conformationnelle très importante en raison de l'absence de chaîne latérale dans cet acide aminé. La sérine et la thréonine sont normalement hydrophiles, mais on peut aussi les rencontrer dans des environnements hydrophobes, tels que les hélices α, dans lesquels leur groupe hydroxyle perd leur caractère hydrophile du fait de la liaison hydrogène formée avec le groupe carbonyle de la chaîne principale. Au sein des feuillets β hydrophobes, la thréonine est susceptible de remplacer parfois des résidus hydrophobes, grâce au groupe méthyle de sa chaîne latérale.

On peut distinguer quatre groupes d'acides aminés selon leur hydrophobicité :

(i) - les résidus fortement hydrophobes : V, I, L et F,
(ii) - les résidus moyennement hydrophobes : W, M et Y,

→ W apparait à des sites de surface plus souvent que F,
→ M est rencontré à des sites divers, internes ou non ;
→ Y peut s'accommoder des environnements hydrophobes internes et est souvent trouvé dans des boucles ;

(iii) - les résidus peu hydrophobes : A et C sont quasiment insensibles au caractère hydrophobe de leur environnement ;
(iv) - les résidus hydrophiles : D, E, N, Q, H, K et R.

Grâce à cette méthode HCA, la Demanderesse a trouvé que des protéines qui ont un score HCA avec la iota-carraghénase *d'Alteromonas fortis* supérieur ou égal à 65 % sur le domaine s'étendant entre les acides aminés 164-311 de ladite iota-carraghénase sont des enzymes de type glycosyle hydrolase et plus particulièrement des iota-carraghénases appropriées pour la production d'oligo-iota-carraghénanes à partir de carraghénanes.

Les protéines qui ont un score HCA avec la kappa-carraghénase *d'Alteromonas carrageenovora* supérieur ou égal à 75 % sur le domaine s'étendant entre les acides aminés 117 et 262 de ladite kappa-carraghénase sont des enzymes de type glycosyle hydrolase, et plus particulièrement des kappa-carraghénases appropriées pour la production d'oligo-kappa-carraghénanes à partir de carraghénanes.

Les protéines qui ont un score HCA supérieur ou égal à 80 %, de préférence supérieur ou égal à 85 %, avec le domaine 117-262 ci-dessus, sont particulièrement préférés aux fins de l'invention.

Les protéines ci-dessus sont avantageusement extraites de bactéries marines.

Un exemple particulier de glycosyle hydrolase obtenue avec un gène selon l'invention est la protéine ayant la séquence en acides aminés [SEQ ID n°2] extraite *d'Alteromonas carrageenovora.*

Un autre exemple particulier de glycosyle hydrolase obtenue avec un gène selon l'invention est la protéine ayant la séquence en acides aminés [SEQ ID n°4] extraite de *Cytophaga drobachiensis.*

Comme indiqué précédemment, les gènes codant pour les glycosyle hydrolases selon l'invention peuvent être obtenus par séquençage du patrimoine génétique des bactéries qui produisent les glycosyle hydrolases, telles que définies ci-dessus, selon les méthodes classiques bien connues de l'homme du métier.

L'invention concerne également les vecteurs d'expression qui portent, avec les moyens pour leur expression, les séquences nucléiques selon l'invention.

Ces vecteurs d'expression peuvent être utilisés pour transformer des microorganismes procaryotes, en particulier *Escherichia coli* ou des cellules eucaryotes, telles que des levures ou des champignons.

L'invention va être maintenant décrite plus en détail par les exemples illustratifs et non limitatifs ci-après.

Les méthodes utilisées dans ces exemples sont des méthodes, bien connues de l'homme du métier, qui sont décrites en détail dans l'ouvrage de Sambrook, Fristsch et Maniatis intitulé "Molecular cloning : a laboratory manual" publié en 1989 par les éditions Cold Spring Harbor Press à New-York (2ème édition).

La description ci-après sera mieux comprise à l'aide des figures 1 à 2 qui représentent respectivement :

Fig. 1 : L'alignement d'après la similitude maximale selon la méthode de Needleman et Wunsch 1970, J. Mol. Biol. 48, 443-453 de la séquence en acides aminés de la kappa-carraghénase *d'Alteromonas carrageenovora* (partie supérieure) et de *Cytophaga drobachiensis* (partie inférieure).

Fig. 2 : Les profils HCA des séquences en acides aminés des kappa-carraghénases de *Cytophaga drobachiensis* et *d'Alteromonas fortis.*

Les abréviations ou symboles spéciaux utilisés dans les exemples ci-après pour les acides aminés sont les suivants :

Glycine : ◊
Proline : *
Thréonine : □
Sérine : ⊡
Alanine : A
Valine : V
Leucine : L
Isoleucine : I
Méthionine : M
Phénylalanine : F
Tryptophane : W
Cystéine : C
Asparagine : N
Glutamine : Q
Tyrosine : Y
Aspartate : D
Glutamate : E
Lysine : K
Arginine : R
Histidine : H

**EXEMPLE :**

**Les kappa-carraghénases *d'Alteromonas carrageenovora* et de *Cytophaga drobachiensis***

**<u>SECTION 1</u> : Clonage des gènes des kappa-carraghénases**

*Alteromonas carrageenovora* ATCC 43555 a été obtenue auprès de l'American Type Culture Collection. Les souches *A. carrageenovora* et *C. drobachiensis* ont été cultivées sur du milieu de Zobell à 25°C.

On a constitué des banques génomiques d'ADN de *C. drobachiensis* et d'*A. carrageenovora.*

La souche utilisée pour réaliser ces banques, qui est *Escherichia coli* DH5α (Rec A, *endA*1, *gyrA*96, *thil*, *hsdR*17 [rk- mk+], *supE*44, *relA*1, *lac*ZΔM15) a été cultivée sur milieu de Luria-Bertani (milieu LB) à 37°C ou sur un milieu appelé Zd (Bacto tryptone 5 g/l, extrait de levure 1 g/l, NaCl 10 g/l ; pH = 7,2) à 22°C auquel on a ajouté 2 % de κ-carraghénane.

Les milieux de culture gélosés ou non ont été additionnés d'ampicilline (50 μg/ml) ou de tétracycline (15 μg/ml), à partir de solutions stock préparées dans 50 % d'éthanol (pour éviter la prise en masse à la température de stockage, - 20°C) sauf pour la souche DH5α non recombinante.

Le vecteur d'expression utilisé est le plasmide pAT153 décrit dans Nature 283 : 216 (1980). Ce plasmide contient deux gènes de résistance aux antibiotiques : un gène de résistance à la tétracycline et un gène qui code pour une β-lactamase, enzyme de la membrane cytoplasmique qui dégrade l'ampicilline.

L'ADN total de *C. drobachiensis* et l'ADN total de *A. carrageenovora* ont été préparés selon le mode décrit par Barbeyron et al. [J. Bacteriol. 160, 586-590 (1984)].

Les ADN génomiques de *C. drobachiensis* et *A. carrageenovora* ont été coupés avec les endonucléases de restriction, respectivement *Nde*II et *Sau*3AI. En effet, dans le cas de *C. drobachiensis,* l'endonucléase de restriction *Nde*II a été utilisée de façon préférentielle du fait que l'ADN de cette bactérie est méthylé sur le résidu C de la séquence GATC.

Les fragments purifiés d'ADN de 5 000 à 10 000 pb ont été clones sur le site *Bam*HI du plasmide pAT153, qui coupe le gène de résistance à la tétracycline.

Dans chacune des banques génomiques, 6 000 clones ont été obtenus.

### 1. Clonage à partir *d'Alteromonas carrageenovora*

L'obtention de ce gène est décrite en détail par T. Barbeyron dans la Thèse de Doctorat de l'Université Pierre et Marie Curie, Roscoff, soutenue le 28 octobre 1993 et dans Gene 139, 105-109 (1994).

A partir de la banque génomique *d'Alteromonas carrageenovora,* 4 clones d'*E. coli* appelés K1 à K4 ont été capables d'hydrolyser le kappa-carraghénane.

Les plasmides pKA1 à pKA4 ont été purifiés à partir des quatre clones indépendants et cartographiés à l'aide des endonucléases de restriction *Bam*HI, *Dra*I, *Eco*RI, *Hind*III, *Mlu*I, *Pst*I, *Pvu*II, *Sal*I, *Ssp*I, *Xba*I et *Xho*I.

Dans chaque plasmide, on a remarqué la présence d'un fragment *Dra*I-*Hind*III de 2,2 kb.

Ce fragment commun, qui est l'insert entier du plasmide pKA3, a été entièrement séquence à partir du plasmide pKA3.

### 2. Clonage à partir de *Cytophaga drobachiensis*

A partir de la banque génomique de *C. drobachiensis,* cinq clones de *E. coli* appelés pKC1 à pKC5 ont été capables de creuser un trou dans le substrat. Les plasmides isolés et purifiés à partir desdits clones ont été cartographiés avec des endonucléases de restriction.

Des fragments internes de 1 100 pb et 600 pb, respectivement ont été sous-clonés à partir de pKC1 dans le phagemide pbluescript et ont été appelés pKCE11 et pKCN6.

Les plasmides pKC 1, pKCE11 et pKCN6 ont été utilisés pour déterminer la séquence nucléotique du gène de la kappa-carraghénase.

## SECTION 2 :Détermination des séquences des gènes codant pour les kappa-carraghénases *d'Alteromonas carrageenovora* et de *Cytophaga drobachiensis*

### 1. Séquence du gène *d'Alteromonas carrageenovora*

Le nombre de nucléotides de l'insert de pKA3, est de 2 180 pb. La traduction dans les six cadres de lecture révèle la présence de trois phases ouvertes, dont une seule est complète, séparant les deux autres qui ne sont que partielles. Elles sont toutes les trois localisées sur le même brin d'ADN. La seconde phase ouverte appelée *cgkA,* lue dans le troisième cadre de lecture, contient 1 191 pb [SEQ ID N°1].

Le produit de traduction du gène *cgkA* correspond à une protéine de 397 acides aminés pour une masse moléculaire théorique de 44 212 Da (SEQ ID N°2). Le profil hydropathique de cette protéine montre à l'extrémité N-terminale un domaine hautement hydrophobe qui s'étend sur 25 acides aminés. Ce domaine comprend un acide aminé chargé positivement (Lys), suivi d'un segment riche en acides aminés hydrophobes, puis de trois acides aminés polaires. Ces résultats suggèrent qu'il s'agit là d'un peptide signal. La séquence N-terminale de la protéine purifiée à partir du surnageant de culture a été déterminée, confirmant ainsi l'identité du gène. Ces résultats indiquent que la signal peptidase coupe la protéine entre les résidus 25 et 26, en accord avec la règle (-3, -1) de Von Heijne. La protéine mature a donc une masse moléculaire théorique de 41,6 kDa.

## 2. Séquence du gène de *Cytophaga drobachiensis*

L'insert de pKC 1 de 4 425 pb contient un seul cadre ouvert de lecture de 1 635 pb appelé *cgkA* (SEQ ID N°3).

La protéine traduite à partir du gène de la kappa-carraghénase est une protéine comprenant 545 acides aminés avec une masse moléculaire de 61,466 kDa [SEQ ID N°4]

Le profil hydropathique de cette protéine montre à l'extrémité N-terminale un domaine hautement hydrophobe, suggérant qu'il s'agit là d'un peptide signal.

En accord avec la règle (-3, -1) de Von Heijne, le site de clivage de la signal peptidase doit se situer entre la thréonine et la sérine aux positions respectives 35 et 36, avec comme codon initiateur le codon $ATG^{875}$.

La masse moléculaire de la protéine calculée après retrait du peptide signal est de 57,4 kDa, supérieure à la masse moléculaire déterminée pour la κ-carraghénase purifiée extracellulaire de 40,0 kDa.

## SECTION 3 : Comparaison des séquences protéiques des κ-carraghénases *d'Alteromonas carrageenovora* et de *Cytophaga drobachiensis*

La κ-carraghénase de *C. drobachiensis* est similaire à 36,1 % sur l'ensemble de l'alignement en séquences linéaires avec la κ-carraghénase *d'Alteromonas carrageenovora.*

Cette similitude est particulièrement forte entre les acides aminés 117 et 262 (51,8 %) (numérotation de la κ-carraghénase d'*Alteromonas carrageenovora)* (Fig. 1).

Cette similitude est soulignée comme précédemment par analyse HCA qui montre un score HCA entre les deux protéines de 75,4 % sur ledit domaine de 145 acides aminés (Fig. 2).

L'analyse HCA montre également que ces deux protéines appartiennent à la famille 16 des glycosyle hydrolases qui comprend des endoxyglucane transférases (XET), des laminarinases, des lichénases et des agarases. En effet, le score HCA des deux kappa-carraghénases avec les XET est de 67,5 %, de 67,6 % avec les laminarinases, de 73,7 % avec les lichénases et de 71,5 % avec les agarases.

## Revendications

1. Gènes qui codent pour des glycosyle hydrolases ayant un score HCA avec la kappa-carraghénase d'*Alteromonas carrageenovora* qui est supérieur ou égal à 75 % sur le domaine s'étendant entre les acides aminés 117 et 262 de la séquence protéique SEQ ID N° 2 de ladite kappa-carraghénase.

2. Gènes selon la revendication 1, **caractérisés en ce que** le score HCA est supérieur ou égal à 80 %.

3. Gènes selon la revendication 1, **caractérisés en ce que** le score HCA est supérieur ou égal à 85 %

4. Gène selon la revendication 1, **caractérisé en ce qu'**il code pour la κ-carraghénase de *Cytophaga drobachiensis* et qu'il comprend la séquence nucléique SEQ ID N° 3.

5. Utilisation des gènes selon l'une quelconque des revendications 1 à 4, pour l'obtention de glycosyle hydrolases par génie génétique.

6. Utilisation du gène selon la revendication 4 pour l'obtention de la kappa-carraghénase de *Cytophaga drobachiensis* par génie génétique.

```
1   MKKPNFYGKMGRTALSSLFYLFFLGLVYGQQPTKTSNPNDQWTIKWSASDEFN_KNDPDW   59
1   MKPISIVAFPIPAISMLLLSAVSQAASM_QPPIAK_PGETWILQAKRSDEFNVK_DAT_   55

60  AKWIK_TGNLPNTSAWKWN_NQKNVKISNGIAELTM_RHNANNTPPDGGT______YF__  108
56  _KWNFQTENYGVWS_WK_NENAT_V__SNGKLKLTTKRESHQRTFWDGCNQQQVANYPLY  109

109 _TSGIFKSYQKFTYGYFEAKIQGADIGEGVCPSFWLYSDFDYSVAN_GETVYSEIDVVEL  166
110 YTSGVAKSRATGNYGYYEARIKGASTFPGVSPAFWMYSTIDRSLTKEGDVQYSEIDVVEL  169

167 QQFDWY_EGHQDDIYDMDLNLHAVVKENGQGVWKRPKMYPQEQLNKWRAM_DPSKDFHIY  224
170 TQKSAVRES__DH__DLH_NI__VVK_NGKPTWMRPGSFPQTNHNGYHLPFDPRNDFHTY  221

225 GCEVNQNEIIWYVDGVE_VARKPNKYWHRPMNVTLSLGLRKPFVKFFDNKNNAINPETDA  283
222 GVNVTKDKITWYVDG_EIVGEKDNLYWHRQMNLTLSQGLRAPHTQW___KCNQFYPSAN_  276

284 K_AREKLSDIPTSMYVDYVRVWEKSAGNTTNPPTSEVGTLKTKGSKLVIDHWDASTGTIS  342
277 KSA_EGF___PTSMEVDYVRTWVKVGNNNSAPGEGQSCPNTFVAVNSVQLSAAKQTLRKG  332

343 AVSNNTKTGQYAGSVNNASIAQIVTLKANTSYKVSAFGKASSPGTSAYLGISKASNNELI  402
333 QSTTLESTVLPNCATNKKVIYSSSNKNVATVNSAGVV_KAKNKGTATITVKTKNKGKIDKL  392

403 SNFEFKTTSYSKGEIEIRTGNVQESYRIWYWSSGQAYCDDFNLVEINSGASQLNENETET  462
393 TIAVN                                                        397

463 ALEKGIHIYPNPYKNGPLTIDFGKPFSGEVQITGLNGRTFLRRNVVDQTSVQLLESKSKF  522

523 KSGLYIVKISGPDGEVSKKILVE                                      545
```

FIG.1

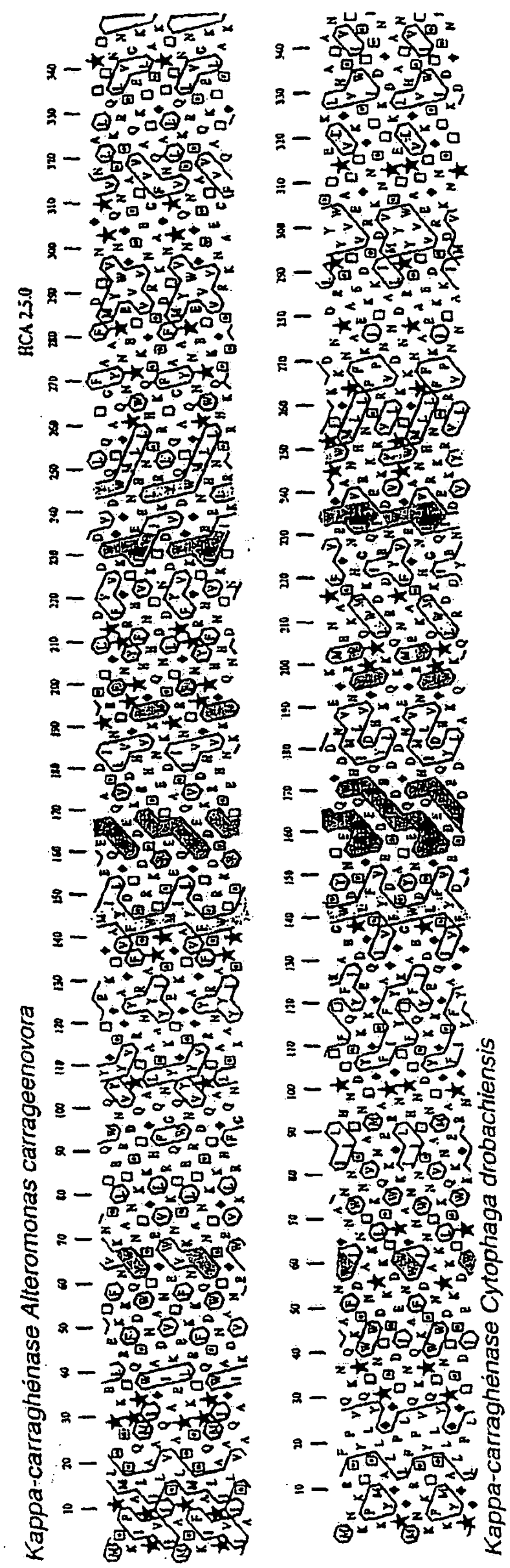
HCA 2.5.0
Kappa-carraghénase Alteromonas carrageenovora
Kappa-carraghénase Cytophaga drobachiensis

FIG.2

Office européen des brevets

# RAPPORT DE RECHERCHE EUROPEENNE

Numéro de la demande
EP 04 29 1327

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,X<br><br><br><br><br><br><br>X | PHILIPPE POTIN (THESIS): "Recherche, production, purification et charactérisation de galactane-hydrolases pour la préparation d'oligosaccharides des parois d'algues rouges."<br>février 1992 (1992-02), UNIVERSITÉ DE BRETAGNE OCCIDENTALE , XP002033462<br>* page 100 - page 103 *<br>* page 110 - page 111 *<br>----- | 1-6<br><br><br><br><br><br><br>1-6 | C12N15/56<br>C12N9/24<br>C12N9/38 |
| D,X | BARBEYRON T ET AL: "THE GENE ENCODING THE KAPPA-CARRAGEENASE OF ALTEROMONAS CARRAGEENOVORA IS RELATED TO BETA-1,3-1,4-GLUCANASES (HYDROPHOBIC CLUSTER ANALYSIS: AGARASE: CARRAGEENASE: BETA-1,3-1,4-GLUCANASE: SEQUENCE COMPARISON)"<br>GENE, ELSEVIER BIOMEDICAL PRESS. AMSTERDAM, NL,<br>vol. 139, 1994, pages 105-109, XP002033461<br>ISSN: 0378-1119<br>* abrégé *<br>* page 105, colonne 2, alinéa 2 *<br>* page 107, colonne 2, alinéa 2 *<br>* page 108, colonne 2, alinéa 2 - page 109, colonne 1, alinéa 2 *<br>----- | 1-6 | DOMAINES TECHNIQUES RECHERCHES (Int.Cl.7)<br>C12N |
| A | GREER C.W. AND YAPHE W.: "Purification and properties of iota-carragenase from a marine bacterium."<br>CANADIAN JOURNAL OF MICROBIOLOGY,<br>vol. 30, 1984, pages 1500-1506,<br>XP000676457<br>* le document en entier *<br>----- | 1-6 | |

Le présent rapport a été établi pour toutes les revendications

| Lieu de la recherche | Date d'achèvement de la recherche | Examinateur |
|---|---|---|
| Munich | 18 août 2004 | Keller, Y |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)